# EUROPEAN PATENT APPLICATION

(11) **EP 1 840 224 A1**
(43) Date of publication of application: **03.10.2007**
(21) Application number: 06075767.1
(22) Date of filing: 29.03.2006
(51) Int. Cl.: C12Q 1/68, A61K 31/337

(54) **Method of predicting survival of a non-small-cell lung cancer patient to a chemotherapeutic treatment**

(71) Applicant: Pangaea Biotech, S.A., 08028 Barcelona (ES)
(72) Inventor: Rosell Costa, Rafael Institut Català d'Oncología, 08916 Badalona (ES); Tarón Roca, Miguel Institut Català d'Oncología, 08916 Badalona (ES)
(74) Representative: ABG Patentes, S.L.

(57) **Abstract**

Method for predicting the survival of a patient suffering from NSCLC to an antimicrotubule agent based chemotherapy treatment which comprises the step of determining the methylation state of a nucleic acid encoding CHFR in a biological sample from the patient, wherein the presence of methylation is indicative of longer survival of said patient as a response to said chemotherapy treatment. The methylation status of the *CHFR* gene can be easily determined in a serum sample.

## Description

### FIELD OF THE INVENTION

The present invention relates to the field of diagnostics, in particular to a method for predicting the survival of non small cell lung carcinoma (NSCLC) patients, and especially of elderly patients, based on the methylation pattern of the gene *CHFR.* It also relates to the use of chemotherapeutic agents selected according to the results of the previous method for the treatment ofNSCLC patients.

### BACKGROUND OF THE INVENTION

Non-small-cell lung cancer (NSCLC) accounts for approximately 80% of all lung cancers, with 1-2 million new cases worldwide each year. NSCLC resulted in more than one million deaths worldwide in 2001 and is the leading cause of cancer-related mortality in both men and women (31% and 25%, respectively).

Treatment of NSCLC is currently based in surgery, whenever possible, and chemotherapy. Cisplatin (DDP) and carboplatin are among the most widely used cytotoxic anticancer drugs, these drugs disrupt DNA structure through formation of intrastrand adducts. Platinum-based chemotherapy regimens have demonstrated to improve survival in patients with advanced NSCLC. However, resistance to these drugs through de novo or induced mechanisms undermines their curative potential. Resistance to platinum agents such as DDP has been attributed to enhanced tolerance to platinum adducts, decreased drug accumulation, or enhanced DNA repair.

Over the last decade newly developed cytotoxic agents including paclitaxel, docetaxel, gemeitabine, and vinorelbine have emerged to offer multiple therapeutic choices for patients with advanced NSCLC. But each of the new regimens can provide only modest survival benefits compared with cisplatin-based therapies. Combination therapies of platinum compounds and these agents provide the bulk of standard treatments at this moment. Stage grouping of the NSCLC patients in TNM subsets (T = primary tumor; N = regional lymph nodes; M = distant metastases) permits the identification of patient groups with similar prognosis and treatment options. The stages are defined as follows:
Stage 1: Cancer is located in only one lung and has not spread to the adjacent lymph nodes or outside the chest.
Stage 11: Cancer is located in one lung and may involve lymph nodes on the same side of the chest but does not include lymph nodes in the space between the lungs (the mediastinum) or outside the chest.
Stage IIIA: Cancer is a single tumor or mass that is not invading any adjacent organs and involves one or more lymph nodes away from the tumor, but not outside the chest.
Stage IIIB: Cancer has spread to more than one area in the chest, but not outside the chest.
Stage IV: Cancer has spread, or metastasized, to different sites in the body, which may include the liver, brain or other organs.

The prognosis of advanced NSCLC is dismal. The overall five-year survival of patients with NSCLC has remained at less than 15% for the past 20 years. Five-year survival is around 25% for pathologic stage IIB (T1-2N1M0, T3N0M0), 13% for stage IIIA (T3N1M0, T1-2-3N2M0), and a low 7% for stage IIIB (T4N0-1-2M0).

A recent Eastern Cooperative Oncology Group trial of 1.155 patients showed no differences among the chemotherapies most used at the moment: cisplatin/paclitaxel, cisplatin/gemcitabine, cisplatin/docetaxel and carboplatin /paclitaxel. Overall median time to progression was 3,6 months, and median survival was 7,9 months, a 1-year survival rate of 33% and a 2-year survival rate of 11 percent (Schiller et al. N Engl J Med 2002 Jan 10; 346(2):92-8). A more recent randomized study of 1218 patients reported a median survival of 11 months in stage IIIB-IV patients.

However, striking differences in survival among patients with advanced disease have been observed, with some surviving years and others only a few months. No clinical parameters have been found that can completely account for these differences.

In view of the above, it is clear that new therapies are urgently needed to improve the response rate, or at least to increase the time to progression and the survival of NSCLC patients.

Another unmet medical need is the provision of tools to select the most appropriate available chemotherapy for each patient. In this sense, one of the most promising advances lies in the field of pharmacogenomics. On one side researchers are trying to find drugs that target patients with a specific genotype (targeted therapy), on the other it provides information to prescribe the existing drugs to specific genotypes (customized therapy).

The identification of specific molecular signatures and genetic polymorphisms that correlate with treatment outcome and treatment-associated toxicity has made it possible to propose "target" populations for cytotoxic therapy in patients with advanced solid tumors and hematologic malignancies. The clinical impact of such an approach can be dramatic. For example, it has bee found that in the target population of lung adenocarcinoma patients harboring specific epidermal growth factor receptor (EGFR) tyrosine kinase domain mutations, treatment with a EGFR tyrosine kinase inhibitors such as gefitinib (tressa TM) can achieve long-lasting responses in a high proportion of patients. Therefore, these mutations may serve as predictors for the reponsiveness to gefitinib, and may be useful in identifying patients likely to benefit from gefitinib therapy. New of such molecular markers are urgently needed.

The *CIIFR* gene (Checkpoint with forkhead and ring finger domains), is a mitotic stress checkpoint gene, that was cloned and localized to chromosome 12q24.33. In mammalian cells exposed to drugs that disrupt microtubule structure, such as nocodazole or paclitaxel, the CHFR protein mediates a delay of entry into metaphase that is characterised microscopically by delayed chromosome condensation- Cell-cycle progression is delayed until the cellular injury has been repaired. In addition, CHFR promotes cell survival in response to mitotic stress.

It was reported that *CHFR* is frequently methylated in cell lines derived from tumors of the colon (80%), brain (100%) and bone (100%). In addition, *CHFR* was methylated in 37% of primary colon adenocarcinomas and in 10% of primary non-small cell lung carcinomas (Corn et al., Carcinogenesis, 2003, vol 24, no. I pp 47-51). Hypermethylation of the *CHFR* gene is associated with silencing of the gene and loss of detectable levels of *CHFR* transcripts, resulting in functional abrogation of the prophase checkpoint (Mizuno K. et al, Oncogene 2002, Apr. 4; 21(15):2328-2333; Toyota et al. PNAS 2003, June 24 vol, 100, no. 13: 7818-7823). Although Corn et al. suggest that the methylation status of *CHFR* may help predict response rates to antimitotic chemotherapies such as the taxanes, following a recent retrospective study with 41 patients, it has been concluded that DNA methylation of *CHFR* is not a predictor of the response to docetaxel and paclitaxel in advanced and recurrent gastric cancer (Yoshida K. et al, Anticancer Research 2006, Jan-Feb, 26(1A):49-54). None of these docuements mentions association of CHFR with time to progression or survival.

While, as explained above, no major overall impact can be attained with traditional chemotherapy in NSCLC, it appears that chemosensitivity and survival are individually predetermined. Nevertheless, in spite of the growing list of genetic abnormalities identified as being involved in DNA repair pathways and altered chemosensitivity in NSCLC patients, translational assays have not yet been developed for use in individualized chemotherapy. Hence, development of standard testing processes available in the clinic is still a key challenge in the field of oncology.

It is an object of the present invention to provide predictors of response to chemotherapy, which can be a valuable clinical tool for use in the selection of optimal treatment modes, in particular for patients suffering from NSCLC, having such a poor survival rate and unpredictable chemosensitivity.

### SUMMARY OF THE INVENTION

The present invention provides a tool for use in predicting differential survival, and tailoring chemotherapy for NSCLC patients.

We have found *CHFR* methylated in the scrum of 30%, of 350 docetaxel/cisplatin and docetaxel/gemcitabine treated stage IV NSCLC patients, which correlate with significant improvement in time to progression and survival. The differences in survival were more striking in the group of patients with performance status 0 and in patients with methylated *CHFR* but unmethylated 14-3-3 sigma. In this subgroup of patients, median survival increased to 33 months, compared with 8 months for patients with unmethylated *CHFR.* Thirty percent of patients were older than 66 years. Surprisingly, in this subgroup of patients with methylated *CHFR,* median survival has not been reached.

Therefore, patients in which *CHFR* is methylated are more likely to benefit from antimicrotubule agents based chemotherapy, such as docetaxel/cisplatin or docetaxel/gemcitabine chemotherapy. In the subgroup of elderly patients having *CHFR* methylated, antimicrotubule agents based chemotherapy such as docetaxel/cisplatin or docetaxel/gemcitabine is very likely to significantly improve survival. On the other hand patients with *CHFR* methylation-negative status can most benefit with chemotherapy not comprising antimicrotubule agents.

In one aspect the invention is directed to an *in vitro* method for predicting the survival following chemotherapy of a patient suffering from nan-small-cell lung cancer (NSCLC) comprising the steps:
a) isolating nucleic acids from a body fluid or tissue sample of the patient;
b) establishing the methylation state of the nucleic acid encoding CHFR in the sample,
c) and classifying the patients in 2 groups defined as methylation-positive or methylation-negative according to the results,
wherein to each group a prognosis relating to survival according to each of the chemotherapeutic alternatives is established.

According to our results, once the methylation the prognosis can be established taking into account that belonging to the methylation-positive group is indicative of longer survival of the patient as a response to an antimicrotubule agent based chemotherapy. This is especially the case for patients above 66 years of age. The antimicrotubule agent based chemotherapy is preferably selected from docetaxel, paclitaxel or vinorelbine as single agentes or a combination selected from docetaxel/cisplatin, paclitaxel/cisplatin, vinorelbine/cisplatin, doectaxel/gemeitabine, vinorelbine/gemeitabine, paclitaxel/gemeitabine, docetaxel/carboplatin, and paclitaxel/carboplatin.
In the above method the nucleic acid is isolated from a tumor sample of the patient, or alternatively from a blood or serum sample of the patient.

In another embodiment the invention is directed to a method for predicting the survival of a patient suffering from NSCLC to a antimicrotubule-based chemotherapy treatment, which comprises the step of determining the methylation state of a nucleic acid encoding *CHFR* in a biological sample from the patient, wherein the cualitative presence of methylation is indicative of longer survival of said patient as a response to said chemotherapy treatment.

In a further embodiment the invention is directed to a method for designing an individual chemotherapy for a patient suffering from NSCLC which comprises:
i) determine the the methylation state of a nucleic acid encoding *CHFR* in a biological sample from the patient;
ii) considering the data obtained in the previous step for designing an individual chemotherapy, wherein the methylation-positive patients are more likely to have a longer survival with a chemotherapy treatment comprising an antimicrotubule agent, and the methylation-negative are more likely to have a longer survival with a chemotherapy not comprising antimicrotubule agent.

In still another embodiment the invention is also directed to the use of an antimicrotubule agent selected from docetaxel, paclitaxel and vinorelbine in the preparation of a medicament for the treatment of NSCLC patient that presents *CHFR* methylation-positive status.

### BRIEF DESCRIPTION OF THE FIGURES

**Figure 1.** Shows the Kaplan-Meier curves for time to progression in NSCLC patients (301) treated with docetaxel/cisplatin or gemcitabine/cisplatin, distributed according to their *CHFR* methylation status.
**Figure 2.** Shows the Kaplan-Meier curves for survival in NSCLC patients (301) treated with doectaxel/cisplatin or gemeitabine/cisplatin, distributed according to their *CHFR* methylation status,
**Figure 3A and 3B.** Shows the Kaplan-Meier curves for time to progression and survival in the subgroup of elderly patientes (Age >66), distributed according to their *CHFR* methylation status.
**Figure 4.** Shows the Kaplan-Meier curves for time to progression in the subgroup of patients responding to chemotherapy (n= 128), distributed according to their *CHFR* methylation status.
**Figure 5.** Shows the Kaplan-Meier curves for survival in the subgroup of patients responding to chemotherapy (n-128), distributed according to their *CHFR* methylation status.

### DETAILED DESCRIPTION OF THE INVENTION

In the present description, the following terms and expressions have the meaning indicated as follow:
"Antimicrotubule agents" means a drug that inhibits cell growth by stopping cell division and used as treatments for cancer. They are also called antimitotic agents, mitotic inhibitors, and taxanes. For example docetaxel and paclitaxel are antimicrotubule agents. The Vinca alkaloids such as vinorelbine, vincristine, vindesine and vinblastine are also antimicrotubule agents. Preferred antimicrotubule agents in the present invention are docetaxel, paclitaxel and vinorelbine.
"Patient that presents *CHFR* methylation-positive status" means that in a cualitative determination of the nucleic acids encoding the gene *CHFR* in a biological sample from as explained below a visible band can be observed in the methylation lane, taking as control full methylated and unmethylated DNA to provide reference lanes.
"Prognosis relating to survival according to each of the chemotherapeutic alternatives" means giving a quantitative evaluation of the risk of a patient dying from NSCLC in a certain period of time, the evaluation being done using as a reference survival curves like those provided in the present description.

As used herein,"a clinical response"is the response of the tumor to treatment with a chemotherapeutic agent. Criteria for determining a response to therapy are widely accepted and enable comparisons of the efficacy alternative treatments. A complete response (or complete remission) is the disappearance of all detectable malignant disease. A partial response is an approximately 50 percent decrease in the product of the greatest perpendicular diameters of one or more lesions, no new lesions and no progression of any lession. A responder is a patient giving a complete or partial response to te esplatin or carbopaltin chemotherapy.

Unless stated otherwise as used herein the term "survival" shall be taken to include all of the following: survival until mortality, also known as overall survival (wherein said mortality may be either irrespective of cause or NSCLC tumor related) ; "recurrence-free survival" (wherein the term recurrence shall include both localized and distant recurrence); metastasis free survival; disease free survival (wherein the term disease shall include NSCLC and diseases associated therewith). The length of said survival may be calculated by reference to a defined start point (c. g., time of diagnosis or start of treatment) and end point (e. g., death, recurrence or metastasis).

In the context of the present invention the term "designing an individual chemotherapy for a subject suffering from NSCLC" is taken to mean the determination of a treatment regimen (i. c., a single therapy or a combination of different therapies that are used for the prevention and/or treatment of the cancer in the patient) for a patient that is started, modified and/or ended based or essentially based or at least partially based on the results of the analysis according to the present invention.

Paclitaxel (Taxol®) is an antimicrotubule agent that promotes the assembly of microtubules from tubulin dimers and stabilizes microtubules by preventing depolymerization. This stability results in the inhibition of the normal dynamic reorganization of the microtubule network that is essential for vital interphase and mitotic cellular functions. In addition, paclitaxel induces abnormal arrays or "bundles" of microtubules throughout the cell cycle and multiple asters of microtubules during mitosis. Paclitaxel, in combination with cisplatin, is indicated for the first-line treatment of non-small cell lung cancer in patients who are not candidates for potentially curative surgery and/or radiation therapy.

Docetaxel is a semisynthetic antineoplastic agent that is very similar to paclitaxel in structure, mechanism of action, and spectrum of antitumor activity. Docetaxel (Taxotere®) is used to treat breast, head and neck, lung, ovarian, prostate, and many other types of cancer. Docetaxel as a single agent has shown response rates of 21-38% in previously untreated patients with non-small cell lung cancer (NSCLC). Combinations of docetaxel with gemeitabine, vinorelbine, ifosfamide, or carboplatin have been effective in the treatment of NSGLC.

The Vinca alkaloids are a subset of drugs that are derived from the periwinkle plant, *Catharanthus roseus* (also *Vinca rosea, Lochnera rosea,* and *Ammocallis rosea*). These compounds act by binding to the tubulin monomers and inhibiting polymerization. They act differently from the taxanes. There are four of them in clinical use: vinorelbine, vineristine, vindesine and vinblastine.

Cisplatin is still the scaffolding of combination chemotherapy in non-small cell lung cancer (NSCLC). As explained above, results tend to be similar whether the partner drug is paclitaxel, docetaxel, or gemeitabine. Similar results are generally obtained with carboplatin, although in a randomized study, median survival was 8.2 months in the paclitaxel/carboplatin arm and 9.8 months in the paclitaxel/cisplatin arm.

Although cisplatin and carboplatin are widely used for NSCLC patients, resistance to these drugs through de novo or induced mechanisms undermines their curative potential. In general, the genetic mechanisms of cancer chemoresistance are difficult to understand. During the past 30 years medical oncologists have focused to optimise the outcome of cancer patients and it is just now that the new technologies available are allowing to investigate polymorphisms, gene expression levels and gene mutations aimed to predict the impact of a given therapy in different groups of cancer patients to tailor chemotherapy.

To further improve the survival rate in patients with Non-Small-Cell Lung Carcinoma (NSCIrC.), their prognostic classification based on molecular alterations is crucial. Such classification will provide more accurate and useful diagnostic tools and, eventually, a more effective selection of the therapeutic options.

One of the most important alterations involved in carcinogenesis is aberrant promoter methylation. The interest in this field has grown due to the implementation of the methylaton specific PCR (MSP) assay. DNA methylation occurs when cytosine is methylated at position 5, this only appears when direcly followed by the base guanine in the CpG dinucleotide. This modification has important regulatory effects on gene expression predominantly when it involves CpG rich areas (CpG islands). Methylated cytosines in the promoter regions of a gene lead to its inactivation.

Methylation and histone modification have become a focus of recent cancer research, and it has been shown that aberrant CpG island methylation in the promoter region is associated with transcriptionally repressive chromatin. Recent efforts have identified a variety of genes inactivated by methylation or histone deacetylation in human cancers. The detection of hypermethylation in the promoter regions of tumor suppressor genes was first reported in the serum of non-small-cell lung cancer patients. Hypermethylation can be analyzed by the sensitive methylation-specific polymerase chain reaction assay, which can identify one methylated allele in 1000 unmethylated alleles (Herman JG, Graff JR, Myohanen S, Nelkin BD, Baylin SB. Methylation-specific PCR: a novel PCR assay for methylation status of CpG islands. Proc Natl Acad Sci USA 1996; 93:9821-6).

The inventors have now surprisingly found that the methylation status of the gene *CHFR* in NSCLC patients is very effective to predict the survival benefit when treated with antimicrotubule based chemotherapy. This benefit is very significant in elderly patients. This allows the physician to make an informed decision as to a therapeutic regimen most likely to improve survival according to the *CHFR* mehylation status with appropriate risk and benefit trade off to the patient. Based on these findings they have defined the method of the invention in its different embodiments that will be described now in detail.

In one aspect the invention provides an *in vitro* method for predicting the survival following chemotherapy of a patient suffering from non-small-cell lung cancer (NSCLC) comprising the steps:
a) isolating nucleic acids from a body fluid or tissue sample of the patient;
b) establishing the methylation state of the nucleic acid encoding CHFR in the sample,
c) and classifying the patients in 2 groups defined as methylation-positive or methylation-negative according to the results,
wherein to each group a prognosis relating to survival according to each of the chemotherapeutic alternatives is established.

First a tissue sample or body fluid of a patient suffering from NSCLC has been made available. The present method can be applied to any type of tissue or body fluid from a patient provided that there is enough for the methylation status of *CHFR* to be determined.

In one embodiment it is preferable to examine tumor tissue. Preferably this is done prior to the chemotherapy. Tumors or portions thereof are surgically resected from the patient or obtained by routine biopsy. To simplify conservation and handling of the samples, these can be formalin-fixed and paraffin-embedded, this is routine practice in oncology.

However, from the clinical point of view, the obtcntion of tissue samples is limited because of the scarcity of tumor tissue obtained by bronchoscopy, for example in stage IV NSCLC patients. In early stages, sometimes we can benefit from the resected tumor specimens that provide tumor tissue for DNA extraction. But a much better alternative is to use body fluids, such as sputum and scrum, as the sample.

It is known that double-stranded DNA fragments frequently occur in considerable quantities in the serum of cancer patients, with significantly higher levels found in the scrum of patients with metastases. For patients with a tumor load 100 g in size (3 x 10¹⁰ neoplastic cells), it is estimated that 3.3% of the tumor DNA is fed into the circulation daily. In head and neck, small-cell lung and non-small-cell lung cancers, the same microsatellite alterations detected in the tumor were also found in plasma or serum DNA (Sanchez-Cespedes M, Monzo M, Rosell R, et al. Detection of chromosome 3p alterations in serum DNA of non-small-cell lung cancer patients. Ann Oncol 1998;9:113-6; Sozzi G, Musso K, Rateliffe C, Goldstraw P, Pierotti MA, Pastorino U. Detection of microsatellite alterations in plasma DNA of non-small cell lung cancer patients: a prospect for early diagnosis. Clin Cancer Res 1999;5:2689-92).

Genetic analysis has shown that cell-free circulating DNA in plasma or serum of cancer patients shares similar genetic alterations to those described in the corresponding tumor. On one study, a high correlation between methylation of some genes in tumor and serum in glioblastoma patient samples and a good correlation in NSCLC patient samples was found (Ramirez, JL, Tarón, M, ct al. Serum DNA as a tool for cancer patient management, Rocz Akad Med Bialymst. 2003;48:34-41).

Therefore, in a preferred embodiment of the invention it is preferred that the sample is a body fluid from the NSCLC patient selected from blood, plasma or serum. More preferably it is serum. Serum is easily and immediately available from the patient, it suffices to take a blood sample and separate the cells by centrifugation.

The nucleic acids, preferably DNA, are extracted from the sample by procedures known to the skilled person and commercially available such as the QIAmp Blood Mini kit of QIAGEN.
Once the nucleic acid is isolated, the method of the invention includes determining the state of methylation of one or more of those nucleic acids encoding the gene *CHFR.*

The expressions "nucleic acid"or "nucleic acid sequence"as used herein refer to an oligonucleotide, nucleotide, polynucleotide, or to a fragment of any of these, to DNA or RNA of genomic or synthetic origin which may be single-stranded or double-stranded and may represent a sense or antisense strand, peptide nucleic acid (PNA), or to any DNA-like or RNA-like material, natural or synthetic in origin.

Any method for determining the methylation state of the nucleic acids can be used, such as those described in WO 02/27019, US 6,017,704, US 6,331,393 and US5,786,146, Herman JG et al. Proc Natl Acad Sci USA 1996; 93:9821-6; or in the publications cited in the background of the invention, each of which is incorporated herein in its entirety. A preferred method is described in the experimental section of Brandes J et al, Carcinogenesis 2005, vol 26, no 6, pp. 1152-1156. It will be apparent to the person skilled in the art that variations of the methods described in these publications can be used, and other primers, conditions and cualitative or cuantitative evaluations can be done. In a preferred embodiment determining the methylation state of the nucleic acid includes amplifying the nucleic acid by means of oligonucleotide primers that distinguishes between methylated and unmethylated nucleic acids. One of such methods is described in detail in the examples.

Preferably the method for detecting a methylated CpG-containing nucleic acid includes contacting a nucleic acid-containing specimen with an agent that modifies unmethylated cytosine, amplifying the CpG-containing nucleic acid in the specimen by means of CpG-specific oligonucleotide primers, wherein the oligonucleotide primers distinguish between modified methylated and non-methylated nucleic acid and detecting the methylated nucleic acid. The amplification step is optional and although desirable, is not essential. The method relies on the PCR reaction itself to distinguish between modified (e. g., chemically modified) methylated and unmethylated DNA.

The term"modifies"as used herein means the conversion of an unmethylated cytosine to another nucleotide which will facilitate methods to distinguish the unmethylated from the methylated cytosine. Preferably, the agent modifies unmethylated cytosine to uracil. Preferably, the agent used for modifying unmethylated cytosine is sodium bisulfite, however, other agents that similarly modify unmethylated cytosine, but not methylated cytosine can also be used in the method. Sodium bisulfite(NaHSO₃) reacts readily with the 5,6-double bond of cytosine, but poorly with methylated cytosine. Cytosine reacts with the bisulfite ion to form a sulfonate cytosine reaction intermediate that is susceptible to deamination, giving rise to a sulfonate uracil. The sulfonate group can be removed under alkaline conditions, resulting in the formation of uracil. Uracil is recognized as a thymine by Taq polymerase (C→U→T) and therefore upon PCR, the resultant product contains cytosine only at the position where 5-methylcytosine occurs in the starting template DNA (mC→mC→C).

The primers used to determine the methylation state of the *CHFR* gene are preferably from the promoter region.The region between CpG dinucleotides within the *CHFR* gene as disclosed in Brandes J et al, Carcinogenesis 2005, vol 26, no 6, pp. 1152-1156 is especially preferred because of the accuracy of the results obtained. The methylation state can be detemined qualitatively or quantitatively. Well known methods such as fluorescence -based quantitative PCR (using fluorescent primers such as Taqman probes) can be used. Further details can be found for exaample in US 6,331,393.

In a preferred embodiment a qualitative detemination is used, it is quicker and simpler to implement in a lab and the results are accurate enough. In this embodiment primers able to discriminate between the metylated or unmethylated DNA, as explained before, are used for the PCR, and then the resulting DNA is purified and its methylation status determined for example by separation through agarose gel electrophoresis. A simple visual examination (needs previous staining) under UV light allows to classify the sample as methylated when bands are present in the methylated lane or unmethylated when bands are present in the unmethylated lane only. Synthetically methylated and unmethylated DNA are used as controls.

Once the methylation status from a sample is obtained, survival can be predicted in accordance with the results obtained by the inventors, and the most appropriate chemotherapeutic regimen selected. Patients with methylation of *CHFR* will have improved chances of survival if treated with antimicrotubule based chemotherapy when compared with those presenting no methylation.

This predicting tool is very significant for elderly patients (above 66 years of age). In this subgroup the result will be in terms of risk of death as follows: the risk of death for methylated patients is about 0.45 times lower than for unmethylated elderly patients. (Hazard Ratio=0.45; 95% CI:0.24-0.85; p=0.01). Or equivalently, the risk of death for unmethylated patients is about 2.20 times higher than for methylated elderly patients. (Hazard Ratio=2.20; 95% CI:1.17-4.13; p=0.01). These results are based on the data of the inventors, using the regression Cox model.

This is very important because the effectiveness of treating elderly patients with NSCLC is controversial. Since chemotherapy has not been very effective, it has been suggested that no treatment may be better than the toxic side effects of chemotherapy. Some physicians and patients do not elect to use chemotherapy due to their perceived potential intolerance and/or a considered short life expectancy. It is estimated that only 20% of elderly patients with advanced NSCLC ever receive chemotherapy. Researchers at the Dana-Farber Cancer Institute recently analyzed treatment and outcome data of over 6,000 elderly patients with stage IV NSCLC who were treated with chemotherapy and the results were comparable to those achieved in younger patients with NSCLC treated with chemotherapy- These findings suggest that chemotherapy for advanced NSCLC was as effective in elderly patients as it was in younger patients and there is no reason to deny therapy based solely on age- Thus, determination of the methylation. status of *CHFR* in this group of patients can provide an extremely valuable tool for selecting the chemotherapeutic regimen.

Elderly patients belonging to the *CHFR* methylation-positive group will be preferably treated with antimicrotubule agents alone or in combination. Thus these patients are more likely to benefit from a chemotherapy selected from docetaxel, paclitaxel or vinorelbine as single agentes or a combination selected from docetaxel/cisplatin, paclitaxel/cisplatin, vinorelbine/cisplatin, docetaxel/gemeitabine, vinorelbine/gemieitabine, paelitaxel/gemeitabine, docetaxel/carboplatin, and paclitaxel/carboplatin. Those belonging to the the *CHFR* mcthylation-negative group will benefit more from a different chemotherapy.

Following chemotherapy, the prediction can be further improved once it is known if the patient belongs to the "responder" group. If so, the risk of death for the methylated-responder group is about 0.52 times lower than for the unmethylated-responder group (Hazard Ratio-0.52; 95% CI:0.27-0.99; p=0.03). Or equivalently, the risk of death for *CHFR* unmethylated-responders is about 1.94 times higher than for *CHFR* methylated-responders. (Hazard Ratio=1.94; 95% CI:1.02-3.70; p=0.03). Survival time ranges in general can be predicted to be in average about at least 44% longer for *CHFR* methylated patients.

The invention being thus described, practice of the invention is illustrated by the experimental examples provided below. These examples should not be interpreted as limiting the scope of the claims.

### EXAMPLES

A prospective study was carried out to assess *CHFR* methylation in the sera of advanced non-small-cell lung cancer patients treated with docetaxel/cisplatin and docetaxel/gemcitabine, and to correlate methylation status with survival.

### Patients

Patients were considered eligible for the present study if they had stage IV or stage IIIB (with malignant pleural effusion) histologically confirmed non-small-cell lung cancer. Other criteria for eligibility included an Eastern Cooperative Oncology Group (ECOG) performance status of 0 (asymptomatic and fully active) or 1 (symptomatic, fully ambulatory, restricted in physically strenuous activity); age of at least 18 years; adequate hematologic function (hemoglobin at least 9 g per deciliter [5.6 mmol per liter], neutrophil count at least 1500 per cubic millimeter, and platelet count at least 100,000 per cubic millimeter); adequate renal function (serum creatinine less than 1.5 times the upper limit of normal); and adequate liver function (bilirubin not more than 1.5 times the upper limit of normal, aspartate aminotransferase and alanine aminotransferase not more than 5 times the upper limit of normal). Patients with clinically overt brain metastases and those who had received previous chemotherapy were excluded. Patients with ECOG performance status of 2 (symptomatic, ambulatory, capable of self-care, more than 50 percent of waking hours spent out of bed) were also excluded, based on results of previous studies where these patients had a high rate of serious adverse events and poor survival.

The characteristics of the patients is shown in **table I**:

| **CHFR** | **Methylated** | **Unmethylated** | **P** |
|---|---|---|---|
| **ECOG** | | | 0.09 |
| 0 | 41(41.8) | 64(31.5) | |
| 1 | 57(58.2) | 139(68.5) | |

| **Age** | | | 0.20 |
|---|---|---|---|
| <=66 | 67(68.4) | 123(60.6) | |
| >66 | 31(31.6) | 80(39.4) | |

| **Gender** | | | 0.87 |
|---|---|---|---|
| Male | 81(82.7) | 169(83.3) | |
| Female | 17(17.3) | 34(16.7) | |

| **Histology** | | | 0.74 |
|---|---|---|---|
| Adenocarcinoma | 50(51) | 97(47.8) | |
| Large cell | 11(11.2) | 23(11.3) | |
| Squamous | 30(30.6) | 58(28.6) | |
| Bronchoalveolar | 1(1) | 5(2.5) | |
| Others | 6(6.1) | 20(9.9) | |

| **Histology** | | | 0.62 |
|---|---|---|---|
| Adcno | 50(51) | 97(47.8) | |
| No Adeno | 48(49) | 106(52.2) | |

| **Stage Disease** | | | 0.99 |
|---|---|---|---|
| IIB (with pleural | 8(8.2) | 17(8.4) | |
| effusion) | | | |

| IV | 90(91.8) | 90(91.8) | |
|---|---|---|---|
| **Response** | | | 0.60 |
| CR+PR | 39(45.9) | 91(50) | |
| SD+PD | 46(54.1) | 91 (50) | |

Objective responses were evaluated by clinical investigators after the third and sixth treatment cycles by repeating the staging procedures. A complete response (CR) was defined as the disappearance of all known sites of disease; a partial response (PR) was defined as a decrease of 50 percent or more in the sum of the products of the largest perpendicular diameters of measurable lesions, no new lesions, and no progression of any lesion; stable discase (SD) was defined as a decrease of less than 50 percent or an increase of less than 25 percent in the sum of the products of the largest perpendicular diameters of measurable lesions and no new lesions; and progressive disease (PD) was defined as an increase of 25 percent or more in the size of one or more measurable lesions, or a new lesion. For the evaluation of response, patients achieving complete or partial response were considered "responders", and all other patients were considered "non-responders". Time to progression was calculated from the date of enrollment to the date of progression. Survival was calculated from the date of enrollment to the date of death or last clinical follow-up.

### Methylation-specfic polymerase chain reaction analysis of CHFR

Ten milliliters of peripheral blood were collected in clot activator tubes, and serum was separated from cells by centrifugation, Samples were sent to our laboratory (Catalan Institute of Oncology, Barcelona, Spain) for *CHFR* methylation analysis. DNA was extracted from 800 microliters of serum using QIAmp DNA Mini blood kit (Qiagen, Valencia, CA, USA) and resuspended in a final volume of 50 microliters. Paired tumor and scrum DNA from an independent group of 28 surgically resected non-small-cell lung cancer patients was used as control. Tumor genomic DNA was also derived from paraffin-embedded resected tumor tissue obtained by laser capture microdissection (Palm, Oberlensheim, Germany). isolated tumor DNA was incubated with proteinase K, and DNA was extracted with phenol-chloroform and ethanol precipitation. Purified serum or tumor DNA was denatured with sodium hydroxide and modified with sodium bisulfite, which converts unmethylated, but not methylated, cytosines to uracil.

Methylation-specific polymerase chain reaction was performed with primers specific for either methylated or the modified unmethylated DNA spanning the region between CpG dinucleotides. DNA samples were then purified with the Wizard DNA purification resin (Promega, Madison, WI, USA), again treated with sodium hydroxide, precipitated with ethanol, and resuspended in water.

The primers specific for methylated DNA were:
CHFR MET FORWARD: 5'TTT TCG TGA TTC GTA GGC, GAC 3'
CHFR MET REVERSE 5 'GAA ACC GAA AAT AAC CCG CG 3'
And the primers specific for unmethylated DNA were:
CHFR UNMET FORWARD: 5'TTG TAG TTA TTT TTG TGA TTT GTA GGT GAT3'
CHFR UNMET REVERSE 5'TAA AAC AAA ACC AAA AAT AAC CCA CA 3'

Annealing temperature 62°C.

They yielded a 93 bp polymerase chain reaction product. The polymerase chain reaction conditions were as follows: 1 cycle of 95°C for 12 minutes; 45 cycles of 95°C for 30 seconds, 58°C (unmethylated reaction) or 64°C (methylated reaction) for 30 seconds, 72°C for 30 seconds; and 1 cycle of 72°C for 7 minutes.

Placental DNA treated *in vitro* with Sss 1 methyltransferase (New England Biolabs, Beverly, MA, USA) was used as a positive control for methylated alleles of *CHFR,* and DNA from normal lymphocytes was used as a negative control. Ten microliters of each 50-microliter methylation-specific amplified product was loaded directly onto non-denaturing 2 percent agarose gels, stained with ethidium bromide, and examined under ultraviolet illumination. Samples were scored as methylation-positive when methylated alleles were present, visualized as bands in the methylated DNA lane, and as methylation-negative when bands were seen only in the unmethylated DNA lane.

**Results:** The frequency of *CHFR* hypermethylation was 32.6%. There was no association between methylation and performance status (PS), age, gender, histology, response, 14-3-3 sigma serum DNA methylation, polymorphisms in lymphocyte DNA (ERCC1 118 C/T, ERCC1 C8092A, XRCC3 241 ThrMet), or tumor ERCC1 mRNA levels.

Overall, there was a tendency to better median survival for patients with methylated *CHFR.* In patients with performance status 0, median survival was 33 months for 41 patients with methylated *CHFR* and 12 months for 64 patients with unmethylated *CHFR* (P=0.23).

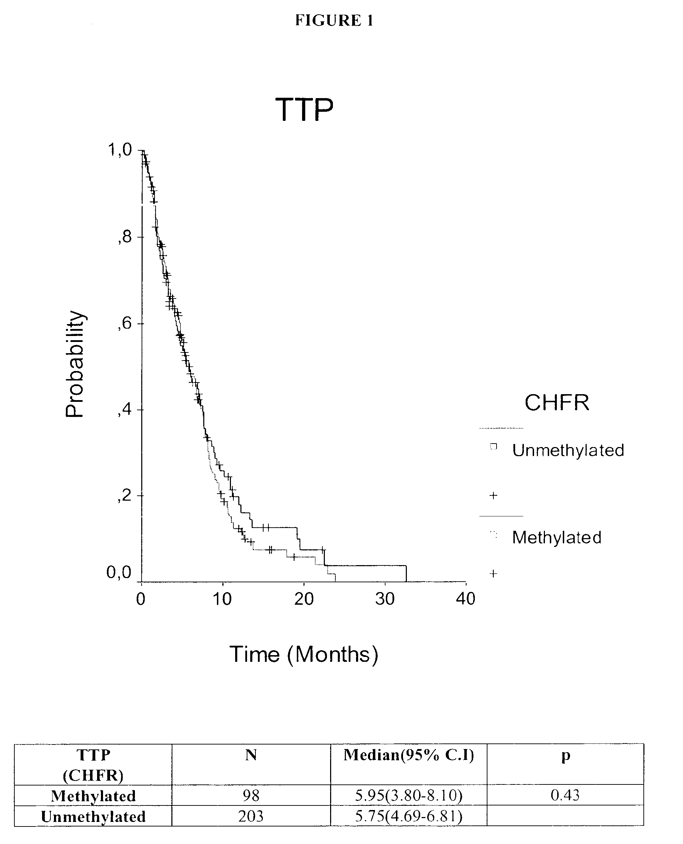

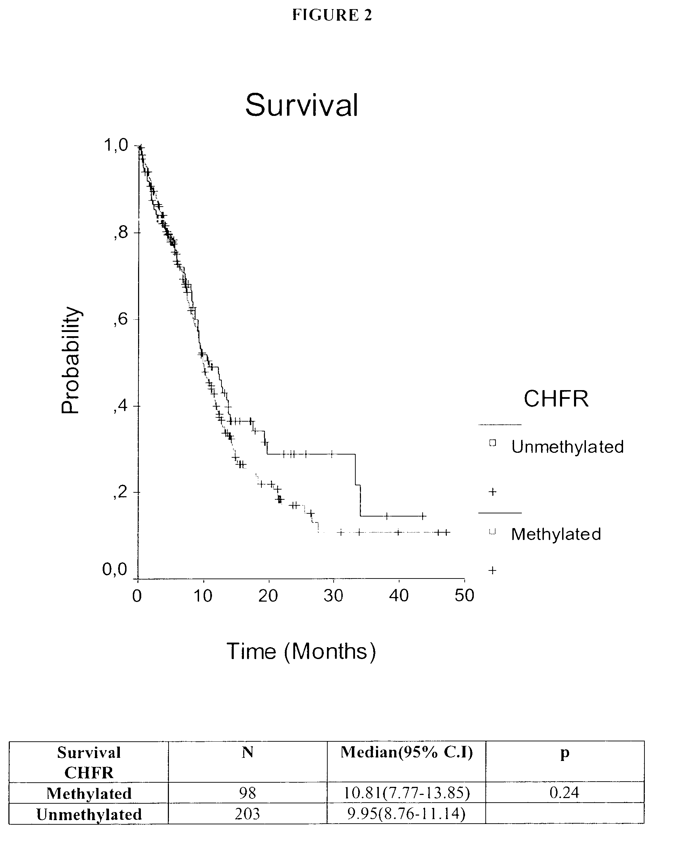

The results are represented in figures 1 and 2, representing the Kaplan-Meier curves for Time to progression and Survival according to the *CHFR* methylation state.

In patients above 66 years of age, median survival was not reached for 31 patients with methylated *CHFR* and was 9.6 months for 80 patients with unmethylated *CHFR* (P-0.01), while in patients under 66 years of age, median survival was 9.4 months for 67 patients with methylated *CHFR* and 10 months for 123 patients with unmethylated *CHFR* (P=0.62).

These results are shown in table 2:

| | **Age** | |
|---|---|---|
| **CHFR** | **<=66** | **>66** |
| **TTP** | | |
| **Methylated** | 67; 5.45(3.93-6.98) | 31; 7.60(4.05-11.15) |
| **Unmethylated** | 123; 5.19(4.44-5.94) | 80; 6.91 (15.61-8.21) |
| | 0.70 | 0.07 |

| **Survival** | | |
|---|---|---|
| **Methylated** | **67; 9.45(7.02-11.89)** | **31; Not Reached** |
| **Unmethylated** | **123; 10.02(8.89-11.14)** | **80; 9.62(7.35-11.89)** |
| | **0.62** | **0.01** |

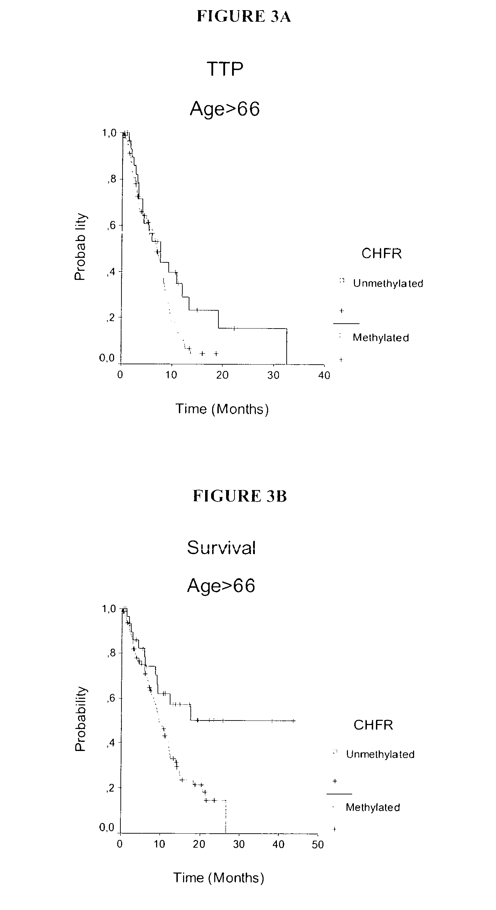

Figure 3A and 3B show these results, whereas the difference in time to progression is not pronounced, the difference in survival is striking.

Patients with both *14-3-3σ* and *CHFR* methylation showed a tendency to longer survival.

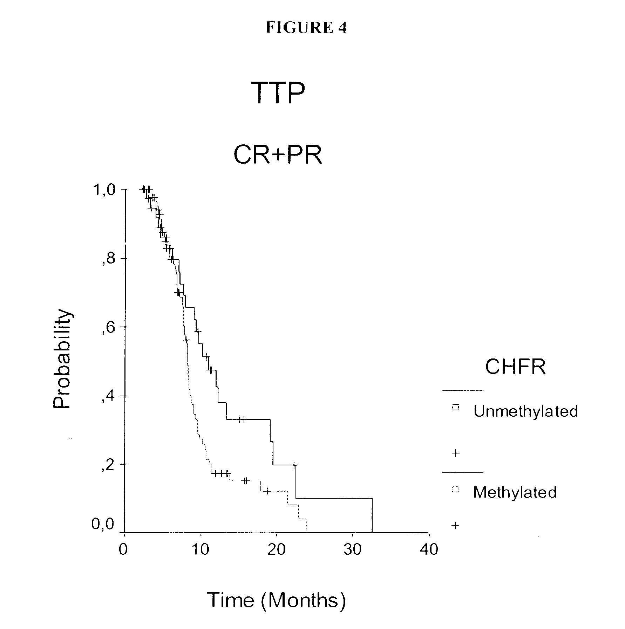

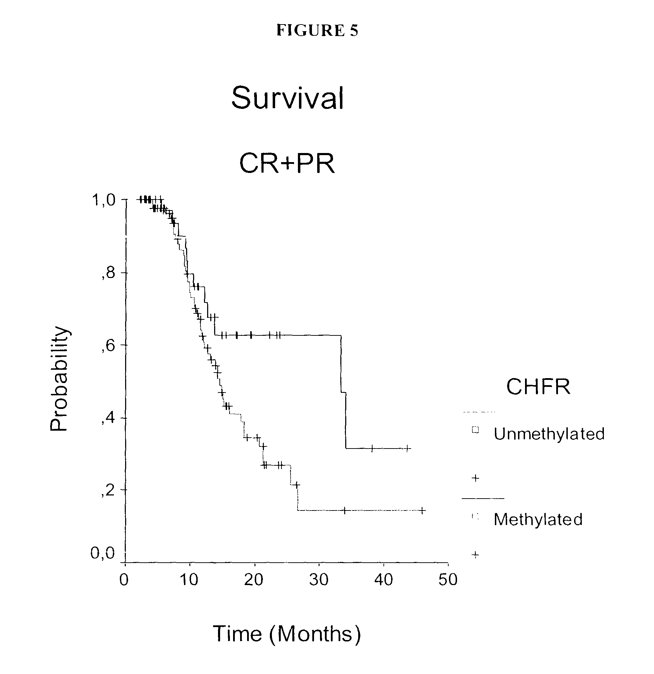

Further, we found that in the subgroup of responders (patients with a complete or partial response following chemotherapy) the difference in survival for *CHFR* methylated patients is bigger, the probability of survival being significantly much higher for methylated than for unmethylated patients. The results are shown in table 3 and 4 and figures 4 and 5:

**Table 3:**

| **TTP (C:R+PR) (Landmark)** | N | Median (95% CI) | p |
|---|---|---|---|
| Methylated | 38 | 10.88(7.67-14.09) | **0.03** |
| Unmethylated | 90 | 8.17(7.70-8.63) | |

**Table 4:**

| **Survival (CR+PR) (Landmark)** | N | Median (95% CI) | P |
|---|---|---|---|
| Methylated | 38 | 33.19(13.77-52.61) | **0.04** |
| Unmethylated | 90 | 14.58(12.59-16.57) | |

## Claims

1. An *in vitro* method for predicting the survival following chemotherapy of a patient suffering from non-small-cell lung cancer (NSCLC) comprising the steps:
a) isolating nucleic acids from a body fluid or tissue sample of the patient;
b) establishing the methylation state of the nucleic acid encoding CHFR in the sample,
c) and classifying the patients in 2 groups defined as methylation-positive or methylation-negative according to the results,
wherein to each group a prognosis relating to survival according to each of the chemotherapeutic alternatives is established.

2. A method according to claim 1, wherein belonging to the methylation-positive group is indicative of longer survival of said patient as a response to an antimicrotubule agent based chemotherapy.

3. A method according to claims 1 or 2, wherein the patient is above 66 years of age.

4. A method according to any of claims 1-3, wherein the state of methylation of the nucleic acid is determined in the regulatory region of the nucleic acid.

5. A method according to claim 4, wherein the regulatory region is the promoter region of the CHFR gene, preferably in CpG rich promoter regions.

6. A method according to any of claims 1-5, wherein the nucleic acid is isolated from a tumor sample of the patient.

7. A method accoding to any of claims 1-5, wherein the nucleic acid is isolated from a blood or serum sample of the patient.

8. The method according to claim 1 wherein the antimicrotubule agent based chemotherapy is selected from docetaxel, paclitaxel or vinorelbine as single agentes or a combination selected from docetaxel/cisplatin, paclitaxel/cisplatin, vinorelbine/eislilatin, doectaxel/gemeitabine, vinorelbine/gcmcitabine, paclitaxel/gemcitabine, docctaxcl/carboplatin, and paclitaxel/carboplatin.

9. Method for predicting the survival of a patient suffering from NSCLC to a antimicrotubule-based chemotherapy treatment which comprises the step of determining the methylation state of a nucleic acid encoding *CHFR* in a biological sample from the patient, wherein the cualitative presence of methylation is indicative of longer survival of said patient as a response to said chemotherapy treatment.

10. Method according to claim 9, wherein the patient is above 66 years of age.

11. Method for designing an individual chemotherapy for a patient suffering from NSCLC which comprises:
i) determine the the methylation state of a nucleic acid encoding *CHFR* in a biological sample from the patient;
ii) considering the data obtained in the previous step for designing an individual chemotherapy, wherein the methylation-positive patients are more likely to have a longer survival with a chemotherapy treatment comprising an antimicrotubule agent, and the methylation-negative are more likely to have a longer survival with a chemotherapy not comprising antimicrotubule agent.

12. The method according to claim 11 wherein the patient is above 66 years of age.

13. Use of an antimicrotubule agent selected from docetaxel, paclitaxel and vinorelbine in the preparation of a medicament for the treatment of NSCLC patient that presents *CHFR* methylation-positive status.


SEQUENCE LISTING

<110> FUNDACIÓN CÁNCER DE PULMÓN

<120> METHOD OF PREDICTING SURVIVAL OF A NON-SMALL-CELL LUNG CANCER
PATIENT TO A CHEMOTHERAPEUTIC TREATMENT

<140> EP06075767.1
<141> 2006-03-29

<160> 4

<210> 1
<211> 21
<212> DNA
<213> Artificial sequence

<220> Synthetic DNA
<223> Primer CHFR MET FORWARD specific for methylated DNA

<400> 1
ttttcgtgat tcgtaggcga c                                                21

<210> 2
<211> 20
<212> DNA
<213> Artificial sequence

<220> Synthetic DNA
<223> Primer CHFR MET REVERSE specific for methylated DNA

<400> 2
gaaaccgaaa ataacccgcg                                                  20

<210> 3
<211> 30
<212> DNA
<213> Artificial sequence

<220> Synthetic DNA
<223> Primer CHFR UNMET FORWARD specific for unmethylated DNA Assay

<400> 3
ttgtagttat ttttgtgatt tgtaggtgat                                       30

<210> 4
<211> 26
<212> DNA
<213> Artificial sequence

<220> Synthetic DNA
<223> Primer CHFR UNMET REVERSE specific for unmethylated DNA Assay

<400> 4

taaaacaaaa ccaaaaataa cccaca 26